# FASCICULE DE BREVET EUROPEEN

(11) **EP 3 370 620 B1**
(45) Date de publication et mention de la délivrance du brevet: **08.01.2020**
(21) Numéro de dépôt: 16806256.0
(22) Date de dépôt: 07.11.2016
(51) Int. Cl.: A61B 6/10, G21F 3/00

(54) **PARAVENT DE RADIOPROTECTION**
STRAHLENSCHUTZSCHIRM
RADIATION PROTECTION SCREEN

(30) Priorité: 05.11.2015 FR 1560606
(43) Date de publication de la demande: 12.09.2018
(73) Titulaire: Arcita, 34070 Montpellier (FR)
(72) Inventeur: VALADE, Frédéric, 34000 Montpellier (FR); COURTAIS, Hugues, 34090 Montpellier (FR)
(74) Mandataire: Rhein, Alain
(86) Numéro de dépôt international: PCT/FR2016/052881
(87) Numéro de publication internationale: WO 2017/077259

(56) Documents cités:
- WO-A2-2009/156660
- JP-A- 2013 007 640
- JP-U- S5 757 811
- US-A1- 2005 173 658

## Description

La présente invention concerne un paravent de radioprotection.

Typiquement un paravent de radioprotection est utilisé pour séparer une personne d'un élément émettant des rayons ionisants, et pour protéger cette personne de ces rayons.

A cet effet, un paravent de radioprotection, comprend un panneau revêtu d'une feuille de radioprotection (typiquement une feuille plombée).

Cependant la rigidité du paravent ne vient que du panneau, et rien ne peut être fixé à celui-ci afin de ne pas perforer la feuille de radioprotection. De plus, dans le cas de paravent mobile, il n'y a aucune radioprotection au niveau des roues dont la hauteur est en général égale à environ 15 cm.

Les documents US 2005/173658 A1, WO 2009/156660 A2, JP 2013 007640 A et JP S57 57811 U divulguent des paravents de radioprotection selon l'art antérieur comprenant en particulier deux faces principales et une ossature qui rigidifie le paravent et qui porte deux pans.

L'invention résout les problèmes précités, et notamment permet la fixation d'objet au paravent sans altérer la feuille de radioprotection, avec un paravent de radioprotection comme défini dans la revendication 1, des modes de réalisation particuliers étant définis dans le revendications dépendantes.

D'autres particularités et avantages de la présente invention apparaitront dans la description détaillée de deux exemples donnés à titre non limitatifs et illustrés dans les dessins annexés dans lesquels :
- la figure 1 est une vue en perspective d'un premier exemple d'un paravent conforme à la présente invention ;
- la figure 2 est une vue en perspective de l'ossature du paravent de la figure 1, ossature au bas de laquelle est fixée une plinthe ;
- la figure 3 est une vue en perspective d'un second exemple d'un paravent conforme à la présente invention ;

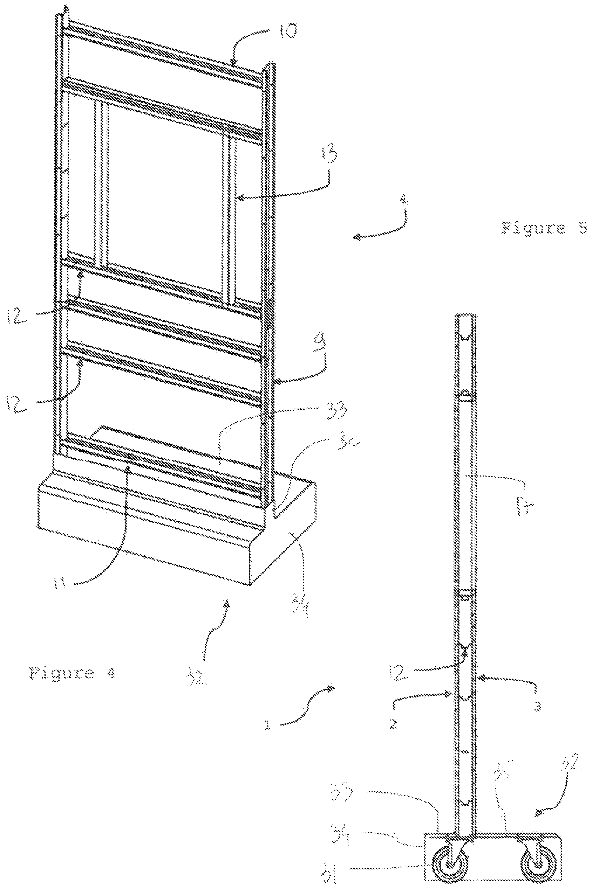

- la figure 4 est une vue en perspective de l'ossature du paravent de la figure 3, ossature au bas de laquelle est fixée une plinthe et un socle ;
- la figure 5 est une vue en coupe verticale passant par le vitrage de protection du paravent de la figure 3 ;

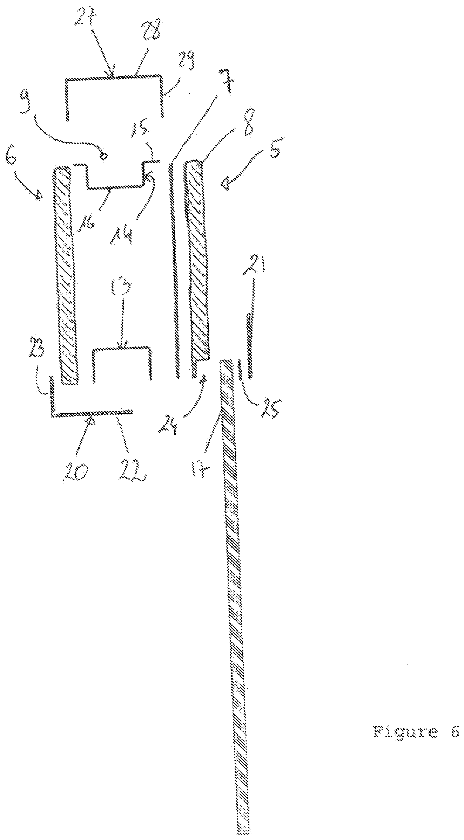

- la figure 6 est une vue en coupe de la partie du paravent situé au niveau des parcloses ;

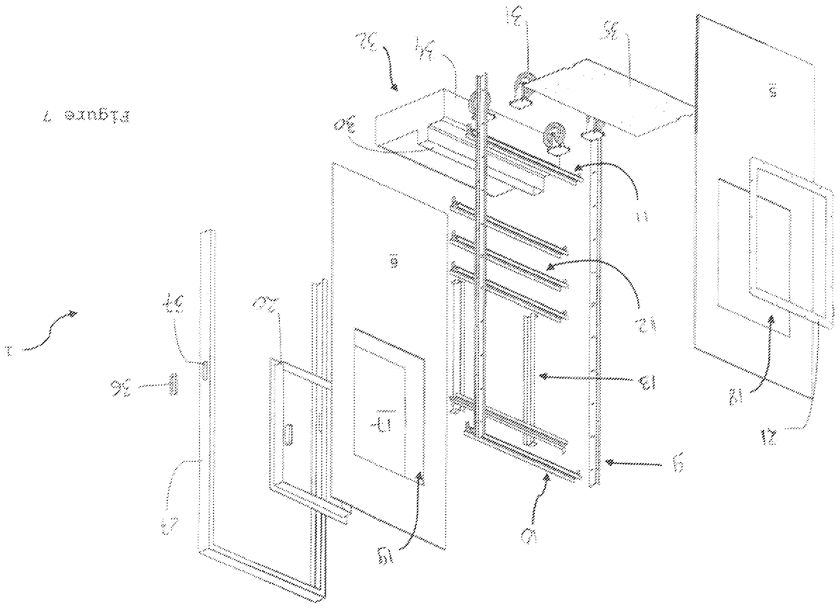

- la figure 7 est une vue en perspective éclatés du paravent de la figuré 3 ;

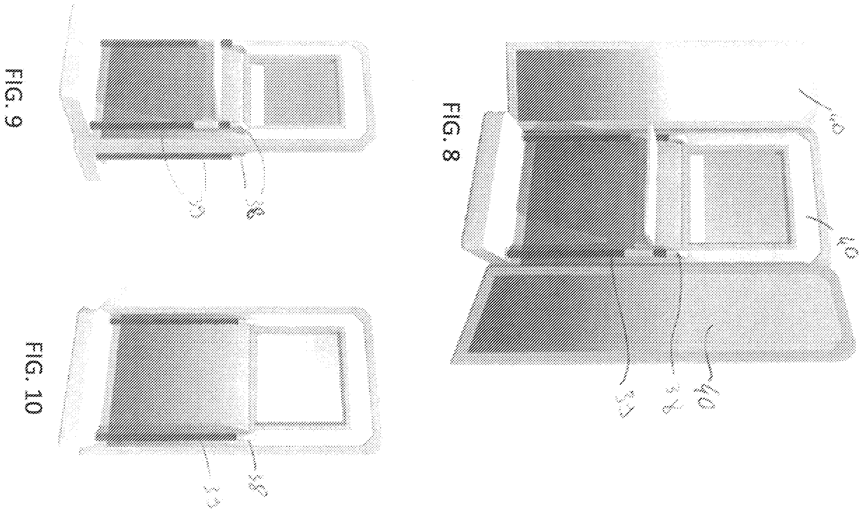

- la figure 8 est une vue en perspective d'un système selon une variante de l'invention ; et
- les figures 9 et 10 sont des vues en perspectives d'un paravent central d'un système selon l'invention.

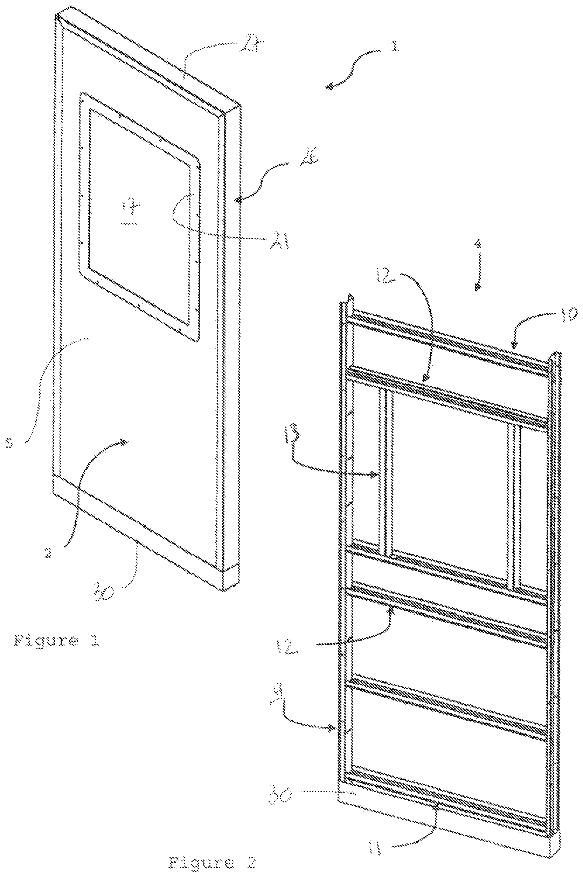

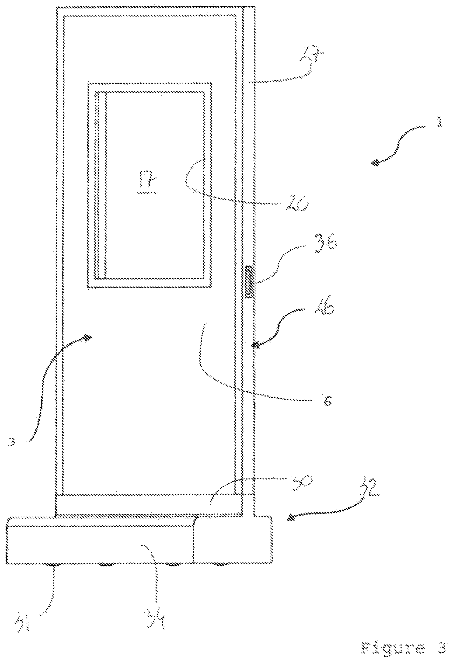

Les figures 1 et 3 représentent en perspective deux exemples de paravent de radioprotection 1 conformes à la présente invention.

Chaque paravent de radioprotection 1 comprend deux faces principales 2, 3 : une face avant 2 destinée à être du côté de la source émettant les rayons ionisants, et une face arrière 3 destinée à être du côté des personnes à protéger des rayons ionisants.

Le paravent de radioprotection 1 comprend une ossature 4 qui le rigidifie et qui porte un pan avant 5 du côté de la face avant 2, et un pan arrière 6 du côté de la face arrière 3. Ici, le pan avant 5 est un pan radioprotecteur et le pan arrière 6 est un pan non radioprotecteur. Dans les présents exemples, chaque paravent de radioprotection 1 présente une hauteur de 2 mètres et une largeur de 0,8 mètre, et la distance séparant la face avant 2 de la face arrière 3 est de 72 millimètres.

Le pan radioprotecteur 5 est formé par une feuille de radioprotection 7 (typiquement une feuille plombée) qui est fixée à l'ossature 4 et un pan non radioprotecteur 8 (typiquement le même pan que le pan arrière 6) qui est fixé à la feuille de radioprotection 7. L'épaisseur de la feuille de radioprotection 7 est d'environ 1 à 2 millimètres.

Le pan non radioprotecteur arrière 6 et le pan non radioprotecteur 8 formant une partie du pan radioprotecteur avant 5 comprennent un panneau intérieur et un panneau extérieur. Le panneau intérieur est fixé à la feuille de plomb 7 (dans le cas du pan avant radioprotecteur 5) ou à l'ossature 4 (dans le cas du pan arrière non radioprotecteur 6). Le panneau extérieur recouvre le panneau intérieur. Typiquement, le panneau intérieur est un panneau en aggloméré (de préférence en aggloméré ayant un bon comportement à l'humidité) et son épaisseur est d'environ de 12 à 16 millimètres. Typiquement, le panneau extérieur est un panneau stratifié et son épaisseur est d'environ 1 à 2 millimètres.

L'ossature 4 est en inox 12-dixièmes et est formée par un assemblage de profilés 9, 10, 11, 12, 13 ce qui confère une solidité et une légèreté au paravent de radioprotection 1 (chaque profilé est en inox).

En particulier, l'ossature 4 comprend des profilés externes 9, 10, 11 et des profilés internes 12, 13. Les pans 5, 6 recouvrent au moins les profilés internes 12, 13. De préférence, les profilés internes sont disposés entre les pans 5, 6.

Chaque profilé 9, 10, 11, 12, 13 a une forme générale en U dont chaque banche latérale porté un pan 5, 6. Ici, la largeur de chaque banche latérale est d'environ 20 millimètres et la largeur de la branche centrale est d'environ 50 millimètres.

L'assemblage de profilés 9, 10, 11, 12, 13 comprend un cadre extérieur rectangulaire formant les limites extérieur du paravent de radioprotection 1. En l'occurrence, ce cadre extérieur comprend deux profilés verticaux 9 et deux profilés horizontaux 10, 11 reliant les deux profilés verticaux 9 (un profilé horizontal supérieur 10 disposé à proximité des extrémités supérieures des deux profilés verticaux 9, et un profilé horizontal inférieur 11 disposé à proximité des extrémités inférieures des deux profilés verticaux 9).

Ici, les profilés verticaux 9 du cadre extérieur ont une forme en U, chaque branche latérale 14 portant un empattement 15 qui est parallèle à la branche centrale 16 et qui est dirigé à l'opposé de l'autre branche latérale 14. La branche centrale 16 est disposée du côté intérieur du paravent de radioprotection 1 et, de ce fait, le chant du cadre extérieur est formé par l'ouverture du U. Chaque empattement 15 a une largeur d'environ 10 millimètres.

L'assemblage de profilés 9, 10, 11, 12, 13 comprend également au moins un profilé horizontal interne 12 qui s'étend de l'un à l'autre des deux profilés verticaux 9 du cadre extérieur (ici, quatre profilés horizontaux internes 12).

Dans les présents exemples, la branche centrale du U de tous les profilés horizontaux 10, 11, 12 (y compris ceux du cadre extérieur) est formée par deux parois horizontales qui sont dans le prolongement de l'une de l'autre de façon à former la majeure partie de la branche centrale, les deux parois horizontales étant reliées l'une à l'autre par un segment de liaison central en forme de U. Le segment central en forme de U comprend une branche centrale et deux branches latérales. La branche centrale du segment de liaison central en U est parallèle aux deux parois horizontales qui forment la majeure partie de la branche centrale du profilé horizontal 10, 11, 12 en U. Les deux branches latérales du segment de liaison central sont parallèles entre eux et avec les deux branches latérales du profilé horizontal 10, 11, 12 en U. Chaque paroi horizontale porte à une première extrémité une branche latérale du profilé horizontal 10, 11, 12 en U qui s'étend selon une première direction, et à une seconde extrémité une branche latérale du segment de liaison central qui s'étend selon une seconde direction opposée à la première direction.

L'assemblage de profilés 9, 10, 11, 12, 13 comprend aussi deux profilés verticaux internes 13 parallèles qui s'étendent entre deux profilés horizontaux internes 12 adjacents. Dans les présents exemples, les deux profilés verticaux internes 13 ont une forme en U et présentent, à chacune de leur extrémité longitudinale, une paroi de liaison qui est portée par la branche centrale du profilé vertical interne 13 et qui est orientée dans la direction opposée à celle des branches latérales du profilé vertical interne 13. Les deux parois de liaison des deux profilés verticaux internes 13 permettent de fixer ces deux profilés 13 aux deux profilés horizontaux internes 12 adjacents. De ce fait, les deux profilés horizontaux internes 12 adjacents et les deux profilés verticaux internes 13 définissent un cadre rectangulaire dans l'ossature 4.

Les pans 5, 6 (plus précisément les panneaux intérieurs des pans 5, 6) sont fixés aux branches latérales des profilés de l'ossature 4 et leurs chants (plus précisément les chants des panneaux intérieurs et extérieurs et de la feuille de radioprotection 7 des pans 5, 6) sont en butée contre les empattements 15 des deux profilés verticaux 9 du cadre extérieur de l'ossature 4.

Le paravent de radioprotection 1 comprend un vitrage de radioprotection 17. Typiquement, ce vitrage de radioprotection 17 est un vitrage dit plombé. Il présente ici une épaisseur de 8,5 millimètres (ce qui correspond à une épaisseur de 2,2 millimètres d'équivalent plomb).

Chaque pan 5, 6 comprend une ouverture 18, 19 située au niveau du vitrage de radioprotection 17. Ici, le vitrage de radioprotection 17 est située au niveau des deux profilés verticaux internes 13 et des deux profilés horizontaux adjacents 12 (plus précisément, les ouvertures en U de ces quatre profilés 12, 13 sont orientés vers les bords du vitrage de radioprotection 17).

Le paravent de radioprotection comprend deux parcloses 20, 21 (ici, en aluminium) qui sont disposées à la jonction du vitrage de radioprotection 17 avec les ouvertures 18, 19 des pans 5, 6 et qui, une fois assemblées, forment une section en U dont la branche centrale est disposée contre et chaque branche latérale ceinture un pan 5, 6 au niveau de son ouverture 18, 19.

Une première parclose 20 a une section droite est en forme de L dont une première barre 22 (celle formant la branche centrale du U des deux parcloses 20, 21 assemblées) ceinture les profilés verticaux internes 13 et les profilés horizontaux adjacents 12 et dont une seconde barre 23 recouvre la bordure périphérique de l'ouverture 18 du pan arrière 6 non radioprotecteur. La première parclose 20 est fixée, d'une part, aux deux profilés verticaux internes 13 et aux deux profilés horizontaux adjacents 12, et, d'autre part, au pan arrière non radioprotecteur 6.

La seconde parclose 21 est un simple encadré qui recouvre la bordure périphérique de l'ouverture 19 du pan avant 5 radioprotecteur. Afin d'assurer une continuité de la radioprotection au niveau du vitrage de radioprotection 17, le panneau intérieur du pan avant radioprotecteur 5 comprend tout le long de la bordure de son ouverture 19, une gorge 24. Dans cette gorge 24 sont disposés le vitrage de radioprotection 17 et une feuille additionnelle de radioprotection 25 (typiquement une feuille plombée). La feuille additionnelle de radioprotection 25 recouvre la partie du vitrage de radioprotection 17 disposée dans la gorge 24. La seconde parclose 21 recouvre entièrement la feuille additionnelle de radioprotection 25 et la partie pleine du panneau intérieur ceinturant la gorge 24. De ce fait, la seconde parclose 21 peut être fixée au pan avant radioprotecteur 5 par vissage sans rompre la continuité de la radioprotection (les vis traversent la seconde parclose 21 et la partie pleine du panneau intérieur qui ceinture la gorge 24).

Le paravent de radioprotection 1 comprend un habillage périphérique 26 (ici, en aluminium) qui forme son chant et sa limite extérieure. L'habillage périphérique 26 recouvre les bordures périphériques extérieures des deux pans 5, 6 et est fixée à l'ossature 4.

Pour chaque bordure périphérique extérieure supérieure et latérale, l'habillage périphérique 26 est un profilé 27 ayant la forme d'un U avec une banche centrale 28 et deux branches latérales 29, chaque banche latérale 29 recouvrant la bordure périphérique extérieure d'un pan 5, 6, et la branche centrale 28 formant le chant du paravent de radioprotection 1.

Pour la bordure périphérique inférieure, l'habillage périphérique 26 est formé par une plinthe 30.

Dans le premier exemple, la plinthe 30 est adaptée à être fixée à un sol de façon à rendre le paravent de radioprotection 1 fixe.

Dans le second exemple, la plinthe 30 est également fixée à des roues 31 de façon à rendre le paravent de radioprotection 1 mobile. Ici, chaque roue 31 est montée pivotante par rapport à la plinthe 30 selon un axe vertical afin de faciliter le changement d'orientation du paravent de radioprotection 1. De la plinthe 30 pend un socle 32 (ici, en inox) qui ceinture les roues 31. Ce socle 32 comprend une paroi supérieure 33 horizontale qui est reliée à la plinthe 30 et à l'ossature 4, et quatre parois latérales 34 verticales qui pendent de la paroi supérieure 33 et qui ceinturent les roues 31. Typiquement, les parois latérales 34 s'étendent de la paroi supérieure 33 jusqu'à une distance proche du sol (environ à 1 centimètre du sol). Dans le second exemple, les roues 31 sont fixées à la face inférieure d'une plaque de fixation 35 dont la face supérieure est fixée à la face inférieure de la paroi supérieure 33.

Dans le second exemple, afin d'assurer la radioprotection au niveau des roues 31, le socle 32 comprend un film de radioprotection (typiquement une feuille plombée). Le film de radioprotection du socle 32 et la feuille de radioprotection du pan radioprotecteur 5 sont agencés de façon à assurer une continuité de la radioprotection. Ici, le film de radioprotection s'étend le long de la partie de la paroi supérieure 33 qui s'étend du côté de la face avant 2, et le long de la paroi latérale 34 qui est située également du côté de la face avant 2. Le film de radioprotection est recouvert par la partie avant de la paroi supérieure 33 et par la paroi latérale 34 avant.

Enfin, afin de pouvoir diriger aisément le déplacement du paravent de radioprotection 1, celui-ci comprend au moins une poignée 36 (ici, deux poignées 36). En l'occurrence, chaque poignée 36 est une poignée creuse qui est disposée dans un orifice 37 réalisé dans la branche centrale de chaque profilé 27 associé à une bordure périphérique extérieure latérale du paravent de radioprotection 1.

On peut aussi prévoir des poignées pleines 38, par exemple tubulaires.

Les poignées 38 comprennent de préférence un matériau antibactérien à leur surface. Par exemple les poignées comprennent une surface métallisée par des métaux ferreux ou non-ferreux et solidifiée à froid.

Outre ses propriétés conférant de la rigidité sans alourdir le paravent de radioprotection, l'ossature 4 permet également de fixer de façon solide un objet au pan arrière non radioprotecteur 6. Cette fixation se fait en prenant, appui sur au moins un profilé 9, 10, 11, 12, 13 de l'ossature 4. L'objet fixé peut ainsi être une tablette horizontale. Afin de limiter l'encombrement, la tablette peut comprendre une première partie fixée à l'ossature 4 et une seconde partie articulée à la première partie. Dans le cas d'un paravent de radioprotection 1 fixe, l'objet fixé peut être un plan de travail (qui peut également prendre appui sur d'autres supports).

Selon une variante, au moins un, de préférence deux bras support 39 peuvent être fixés à l'ossature 4 ou au pan arrière 6. Le bras support 39 peut servir de poignée 38.

Par ailleurs, afin d'éviter un basculement du paravent de radioprotection 1 mobile à cause du poids de l'objet qui y est fixé et du matériel qui y repose, la paroi supérieure 33 du socle 32 s'étend plus du côté de la face arrière 3.

Enfin, la présence de l'ossature 4 permet également d'avoir un paravent de radioprotection 1 comprenant des passages permettant de faire passer des câbles (par exemple des câbles électriques) et d'avoir des prises d'alimentation pour du matériel électrique (notamment celui reposant sur les objets fixés au pan arrière non radioprotecteur 6.

En outre, le paravent peut comprendre au moins un autocollant 40 sur un ou les deux pans. L'autocollant 40 peut permettre de personnaliser le paravent, apposer une marque ou décorer le paravent. De préférence, l'autocollant est antimicrobien.

La divulgation concerne en outre un système comprenant au moins deux paravents accouplés ou susceptibles d'être accouplés ensemble, dont au moins un est tel que décrit précédemment.

De préférence, le système comprend un paravent central portant un ou plusieurs, de préférence deux, paravents latéraux. Les paravents supplémentaires permettent de protéger plus de personnes, en particulier, jusqu'à 4 à 5 personnes avec deux paravents latéraux.

Selon une variante, le paravent central comprend un socle et des roues et de préférence, les paravents latéraux ne comprennent pas de roue.

Selon une variante, les paravents sont accouplés ensemble au moyen d'au moins une charnière. De préférence, la charnière comprend un élément radioprotecteur, ou un pan radioprotecteur comprend une extension recouvrant la charnière.

De préférence, le paravent ou le système comprend un mécanisme configuré pour faire varier la hauteur du paravent ou du système par rapport au sol. Par exemple, une élévation de quelques millimètres permet de faciliter le déplacement tandis qu'un abaissement de sorte à être au contact du sol permet d'améliorer la radioprotection.

## Revendications

1. Paravent de radioprotection (1) comprenant, d'une part, deux faces principales (2, 3), et d'autre part, une ossature (4) qui rigidifie le paravent (1) et qui porte de chaque côté un pan (5, 6), l'un (5) des pans (5, 6) étant radioprotecteur, **caractérisé en ce qu'**il comprend un habillage périphérique (26) qui forme le chant du paravent (1) et recouvre les bordures périphériques des deux pans (5, 6), l'habillage périphérique (26) comprend une plinthe (30) qui recouvre la bordure périphérique inférieure des deux pans (5, 6), la plinthe (30) étant fixée à des roues (31) permettant le déplacement du paravent (1).qui comprend un socle (32) fixé à la plinthe (30), le socle (32) ceinturant les roues (31).

2. Paravent de radioprotection (1) selon la revendication 1, **caractérisé en ce que** l'ossature (4) est formée par un assemblage de profilés (9, 10, 11, 12, 13).

3. Paravent de radioprotection (1) selon la revendication 2, **caractérisé en ce que** chaque profilé (9, 10, 11, 12, 13) à une forme générale en U dont chaque banche latérale porte un pan (5, 6) .

4. Paravent de radioprotection (1) selon l'une des revendications 1 à 3, **caractérisé en ce qu'**il comprend un vitrage de radioprotection (17).

5. Paravent de radioprotection (1) selon l'une des revendications 1 à 4, **caractérisé en ce que** les deux pans (5, 6) sont maintenus parallèles l'un à l'autre par l'ossature (4).

6. Paravent de radioprotection selon l'une des revendications 1 à 5, **caractérisé en ce que** le socle (32) comprend une paroi supérieure (33) horizontale qui est reliée à la plinthe (30) et à l'ossature (4), et quatre parois latérales (34) verticales qui pendent de la paroi supérieure (33) et qui ceinturent les roues (31).

7. Paravent de radioprotection selon l'une des revendications 1 à 6, **caractérisé en ce que** le socle (32) comprend un film de radioprotection.

8. Paravent de radioprotection (1) selon la revendication 7, **caractérisé en ce que** le film de radioprotection du socle (32) et la feuille de radioprotection du pan radioprotecteur (5) sont agencés de façon à assurer une continuité de la radioprotection.

9. Paravent de radioprotection (1) selon l'une des revendications 1 à 8, **caractérisé en ce qu'**une tablette est fixée à l'ossature (4) du côté du pan non radioprotecteur (6).

10. Paravent de radioprotection (1) selon l'une des revendications 1 à 9 dont au moins la revendication 2, **caractérisé en ce que** l'ossature (4) comprend des profilés externes (9, 10, 11) et des profilés internes (12, 13), lesdits pans (5, 6) recouvrant au moins les profilés internes.

11. Système comprenant au moins deux paravents accouplés ou susceptibles d'être accouplés ensemble, dont au moins un est selon l'une des revendications 1 à 10.

12. Système selon la revendication précédente, dans lequel lesdits paravents sont accouplé au moyen d'au moins une charnière.

## Patentansprüche

1. Strahlenschutzschirm (1), der einerseits zwei Hauptflächen (2, 3) und andererseits ein Gerüst (4) umfasst, das den Schirm (1) versteift und auf jeder Seite eine Platte (5, 6) trägt, wobei eine (5) der Platten (5, 6) strahlenschützend ist, **dadurch gekennzeichnet, dass** er eine Umfangsabdeckung (26) umfasst, die den Rand des Schirms (1) bildet und die Umfangsränder der beiden Platten (5, 6) abdeckt, wobei die Umfangsabdeckung (26) eine Sockelleiste (30) umfasst, die den unteren Umfangsrand der beiden Platten (5, 6) abdeckt, wobei die Sockelleiste (30) an Rädern (31) befestigt ist, die das Bewegen des Schirms (1) ermöglichen, der einen Sockel (32) umfasst, der an der Sockelleiste (30) befestigt ist, wobei der Sockel (32) die Räder (31) umgibt.

2. Strahlenschutzschirm (1) nach Anspruch 1, **dadurch gekennzeichnet, dass** das Gerüst (4) durch eine Anordnung von Profilen (9, 10, 11, 12, 13) gebildet ist.

3. Strahlenschutzschirm (1) nach Anspruch 2, **dadurch gekennzeichnet, dass** jedes Profil (9, 10, 11, 12, 13) eine allgemeine U-Form aufweist, wobei jede Seitenwand eine Platte (5, 6) aufweist.

4. Strahlenschutzschirm (1) nach einem der Ansprüche 1 bis 3, **dadurch gekennzeichnet, dass** er eine Strahlenschutzverglasung (17) umfasst.

5. Strahlenschutzschirm (1) nach einem der Ansprüche 1 bis 4, **dadurch gekennzeichnet, dass** die beiden Platten (5, 6) durch das Gerüst (4) parallel zueinander gehalten werden.

6. Strahlenschutzschirm nach einem der Ansprüche 1 bis 5, **dadurch gekennzeichnet, dass** der Sockel (32) eine horizontale obere Wand (33), die mit der Sockelleiste (30) und mit dem Gerüst (4) verbunden ist, und vier vertikale Seitenwände (34), die an der oberen Wand (33) hängen und die Räder (31) umgeben, umfasst.

7. Strahlenschutzschirm nach einem der Ansprüche 1 bis 6, **dadurch gekennzeichnet, dass** der Sockel (32) eine Strahlenschutzfolie aufweist.

8. Strahlenschutzschirm (1) nach Anspruch 7, **dadurch gekennzeichnet, dass** die Strahlenschutzfolie des Sockels (32) und die Strahlenschutzfolie der Strahlenschutzplatte (5) so angeordnet sind, dass ein durchgehender Strahlenschutz gewährleistet ist.

9. Strahlenschutzschirm (1) nach einem der Ansprüche 1 bis 8, **dadurch gekennzeichnet, dass** an der Seite der nicht strahlenschützenden Platte (6) eine Ablage am Gerüst (4) angebracht ist.

10. Strahlenschutzschirm (1) nach einem der Ansprüche 1 bis 9, davon mindestens Anspruch 2, **dadurch gekennzeichnet, dass** das Gerüst (4) Außenprofile (9, 10, 11) und Innenprofile (12, 13) umfasst, wobei die Platten (5, 6) mindestens die Innenprofile abdecken.

11. System mit mindestens zwei miteinander gekoppelten oder koppelbaren Schirmen, von denen mindestens einer einem der Ansprüche 1 bis 10 entspricht.

12. System nach dem vorhergehenden Anspruch, wobei die Schirme über mindestens ein Scharnier gekoppelt sind.

## Claims

1. Radiation protection screen (1) comprising two main faces (2, 3) and a frame (4) which stiffens the screen (1) and supports a panel (5, 6) on each side, one (5) of the panels (5, 6) protecting against radiation, **characterized in that** said screen comprises a peripheral casing (26) which forms the edge surface of the screen (1) and covers the peripheral edges of the two panels (5, 6), the peripheral casing (26) comprises a baseboard (30) which covers the lower peripheral edge of the two panels (5, 6), the baseboard (30) being fixed to wheels (31) which allow the screen (1) to move, which screen comprises a base (32) fixed to the baseboard (30), the base (32) surrounding the wheels (31).

2. Radiation protection screen (1) according to claim 1, **characterized in that** the frame (4) is formed by an assembly of profiles (9, 10, 11, 12, 13).

3. Radiation protection screen (1) according to claim 2, **characterized in that** each profile (9, 10, 11, 12, 13) has a generally U-shaped form, each lateral branch of which supports a panel (5, 6).

4. Radiation protection screen (1) according to any of claims 1 to 3, **characterized in that** it comprises radiation protection glazing (17).

5. Radiation protection screen (1) according to any of claims 1 to 4, **characterized in that** the two panels (5, 6) are held parallel to one other by the frame (4).

6. Radiation protection screen according to any of claims 1 to 5, **characterized in that** the base (32) comprises a horizontal upper wall (33) which is connected to the baseboard (30) and to the frame (4), and four vertical lateral walls (34) which hang from the upper wall (33) and surround the wheels (31).

7. Radiation protection screen according to any of claims 1 to 6, **characterized in that** the base (32) comprises a radiation protection film.

8. Radiation protection screen (1) according to claim 7, **characterized in that** the radiation protection film of the base (32) and the radiation protection sheet of the radiation protection panel (5) are arranged so as to ensure continuity of the radiation protection.

9. Radiation protection screen (1) according to any of claims 1 to 8, **characterized in that** a shelf is attached to the frame (4) on the non-radiation protection panel side (6).

10. Radiation protection screen (1) according to any of claims 1 to 9 including at least claim 2, **characterized in that** the frame (4) comprises external profiles (9, 10, 11) and internal profiles (12, 13), said panels (5, 6) covering at least the internal profiles.

11. System comprising at least two screens which are coupled or can be coupled to one another, at least one of which is a screen according to any of claims 1 to 10.

12. System according to the preceding claim, wherein said screens are coupled by means of at least one hinge.
